# EUROPEAN PATENT SPECIFICATION

(11) **EP 2 533 732 B1**
(45) Date of publication and mention of the grant of the patent: **04.11.2015**
(21) Application number: 11704161.6
(22) Date of filing: 10.02.2011
(51) Int. Cl.: A61F 5/00

(54) **REMOTELY ADJUSTABLE GASTRIC BANDING SYSTEM**
FERNSTEUERBARES MAGENBANDSYSTEM
SYSTÈME DE CERCLAGE GASTRIQUE RÉGLABLE À DISTANCE

(30) Priority: 12.02.2010 US 705245
(43) Date of publication of application: 19.12.2012
(60) Divisional application: 15160040.0
(73) Proprietor: APOLLO ENDOSURGERY, INC., Austin, TX 78746 (US)
(72) Inventor: BIRK, Janel, Oxnard, California 93036 (US); LESLIE, Dustin, Santa Barbara, California 93110 (US); SNOW, Sean, Carpinteria, California 93013 (US); TEZEL, Ahmet, Goleta, California 93117 (US)
(74) Representative: Hoffmann Eitle
(86) International application number: PCT/US2011/024375
(87) International publication number: WO 2011/100450

(56) References cited:
- EP-A1- 2 095 796
- EP-A2- 1 704 833
- WO-A1-2004/014245
- US-A1- 2009 270 904
- US-A1- 2010 010 291

## Description

### FIELD

The present invention generally relates to an implantable device according to the preamble of claim 1.

### BACKGROUND

Adjustable gastric banding apparatus have provided an effective and substantially less invasive alternative to gastric bypass surgery and other conventional surgical weight loss procedures. Despite the positive outcomes of invasive weight loss procedures, such as gastric bypass surgery, it has been recognized that sustained weight loss can be achieved through a laparoscopically-placed gastric band, for example, the LAP-BAND® (Allergan, Inc., Irvine, CA) gastric band or the LAP-BAND AP® (Allergan, Inc., Irvine, CA) gastric band. Generally, gastric bands are placed about the cardia, or upper portion, of a patient's stomach forming a stoma that restricts food's passage into a lower portion of the stomach. When the stoma is of an appropriate size that is restricted by a gastric band, food held in the upper portion of the stomach provides a feeling of satiety or fullness that discourages overeating. Unlike gastric bypass procedures, gastric band apparatus are reversible and require no permanent modification to the gastrointestinal tract.

Over time, a stoma created by a gastric band may need adjustment in order to maintain an appropriate size, which is neither too restrictive nor too passive. Accordingly, prior art gastric band systems provide a subcutaneous fluid access port connected to an expandable or inflatable portion of the gastric band. By adding fluid to or removing fluid from the inflatable portion by means of a hypodermic needle inserted into the access port, the effective size of the gastric band can be adjusted to provide a tighter or looser constriction. Naturally, it would be desirable to allow for non-invasive adjustment of gastric band constriction, for example, without the use of a hypodermic needle.

Birk, et al., US 2010/0010291 A1 is commonly-assigned and copending with the present application. Birk discloses certain approaches to implantable pumping systems that may be relevant. Further US 2009/0270904 A1 discloses a system for facilitating obesity control comprising a gastric banding device including an inflatable member for containing fluid, a fluid reservoir couplable to the inflatable member, an implantable pump unit in communication with said fluid reservoir and effective to control pressure within said inflatable member and including an inductively powered pump and a receiver assembly in communication with the pump.

Some attempts have been made to cause weight loss by deploying an inflatable bladder within the stomach. For example, Brown, U.S. Patent No. 5,259,339 discloses a bladder that may be inflated and deflated within the stomach to control appetite. Brown utilizes a pump that is external to the patient's body. The external pump is connected to the bladder via a percutaneous endoscopic gastronomy tube which creates a permanent channel to the stomach. Brown does not disclose use of the tube in connection with a gastric band.

Some attempts to develop implantable pump systems have used electromagnetic motors to drive the pump systems. For example, Larson, Jr., et al., U.S. Patent No. 5,676,162, discloses a permanent magnet linear electric motor to assist the heart. Such electromagnetic motors can have the drawback of using strong permanent magnets which may affect magnetic resonance (MRI) procedures. Additionally, such motors may lack a gearing system that keeps the piston seal stationary during periods when the electrical source is removed.

Franetzki, et al., U.S. Patent No. 4,883,467, discloses a reciprocating pump for use in an implantable medication dosage device capable of delivering small boluses of drugs that have a volume less than ten micro-liters. Franetzki discloses a device that utilizes an electromagnetic system comprising strong permanent magnets and a high current electromagnet. Also, Franetzki discloses one-way movement of the medication into the patient.

Additionally, Goldowsky, U.S. Patent No. 5,360,445, discloses an implantable blood pump actuator for an artificial heart. The actuator utilizes a voice coil linear electromagnetic motor to power a hydraulic piston.

Some existing pump systems utilize bendable components that are subject to fatigue and failure. For example, Hassler, Jr., et al., U.S. Patent No. 7,374,565 discloses an implantable artificial sphincter system that utilizes a transcutaneous energy transfer for adjustment of the sphincter. A propellant causes a metal bellows to expand or collapse, and the opposite movement is effected by a thermal element that heats or cools the propellant. The repeated expanding and collapsing of the bellows may subject it to fatigue and failure.

Thus, there continues to remain a need for more effective implantable pump systems for use with adjustable gastric bands, particularly such implantable pump systems with increased accuracy in pressure/flow measurements and with increased and more efficient pumping capability.

### SUMMARY

Generally described herein are remotely adjustable and powered gastric banding systems.

The apparatus described herein aid in facilitating obesity control and/or treating obesity-related diseases while being non-invasive once implanted. The invention is defined in claim 1, advantageous embodiments of the invention are subject to the dependent claims.

In one embodiment, an implantable device is configured for filling and draining an inflatable portion of a gastric band. The implantable device comprises a reservoir for holding a fluid and a sensor positioned between the gastric band and the reservoir. The sensor has a first inlet and a second inlet, and the fluid flows from the first inlet to the second inlet when filling the inflatable portion of the gastric band. The fluid flows from the second inlet to the first inlet when draining the inflatable portion of the gastric band. The sensor monitors a parameter of the fluid when filling and draining the inflatable portion of the gastric band.

The implantable device further comprises a first flow control device that is coupled to the first inlet of the sensor. The first flow control device controls a flow of the fluid when filling and draining the inflatable portion of the gastric band. Additionally, the implantable device comprises a pumping device coupled between the reservoir and the first flow control device. The pumping device moves the fluid into and out of the inflatable portion of the gastric band, and the pumping device is a non-magnetic electrical drive capable of being activated and deactivated using a telemetric signal received from a remote device.

### BRIEF DESCRIPTION OF THE DRAWINGS

FIG. 1 illustrates a gastric banding systems.
FIG. 2 illustrates a gastric banding system according to an embodiment of the present invention.
FIG. 3 illustrates a gastric banding system according to another embodiment of the present invention.
FIG. 4 illustrates a cross-sectional view of an implantable pump according to an embodiment of the present invention.
FIG. 5 illustrates a schematic representation of an implantable pump with a flexible bladder according to an embodiment of the present invention.
FIGS. 6A-6B illustrate various configurations of pistons, motors, and leadscrews according to various embodiments of the present invention.
FIGS. 7A-7H illustrate cross-sectional configurations of pistons and leadscrews according to various embodiments of the present invention.
FIG. 8 illustrates a schematic representation of an implantable pump according to an embodiment of the present invention.
FIG. 9 illustrates a schematic representation of an implantable pump with an external reservoir according to an embodiment of the present invention.
FIG. 10 illustrates a schematic representation of an implantable pump with a fluid port in the piston according to an embodiment of the present invention.
FIG. 11 illustrates a schematic representation of an implantable pump within a casing according to an embodiment of the present invention.
FIG. 12A illustrates a schematic representation of an implantable pump within a casing with a volume transfer bladder according to an embodiment of the present invention.
FIG. 12B illustrates a schematic representation of an implantable pump within a casing with a flexible portion according to an embodiment of the present invention.
FIG. 12C illustrates a schematic representation of an implantable pump casing with a bellows according to an embodiment of the present invention.
FIG. 12D illustrates a schematic representation of an implantable pump casing with two bellows according to an embodiment of the present invention.
FIG. 12E illustrates a schematic representation of an implantable pump casing with a slidable portion according to an embodiment of the present invention.
FIG. 12F illustrates a schematic representation of an implantable pump casing with flexible portions according to an embodiment of the present invention.
FIG. 12G illustrates a schematic representation of an implantable pump casing with a bellows and an elongating balloon according to an embodiment of the present invention.
FIGS. 13A-13B illustrate schematic representations of an implantable pump with an external reservoir according to embodiments of the present invention.
FIG. 14 illustrates a cross-sectional view of a multiple piston implantable pump according to an embodiment of the present invention.
FIG. 15 illustrates a cross-sectional view of a multiple piston implantable pump with a bellows according to an embodiment of the present invention.

### DETAILED DESCRIPTION

The present invention generally provides remotely adjustable gastric banding systems, for example, for treatment of obesity and obesity related conditions, as well as systems for controlling inflation of gastric banding systems.

A remotely adjustable gastric band is a medical device which allows a healthcare worker to adjust a gastric band without utilizing hypodermic needles to connect to an implanted access port. An external, handheld controller can be used to send radio frequency signals for powering and communicating with the implanted device. The implanted device can fill or drain the gastric band as requested by the healthcare worker via the handheld controller. The handheld controller may be a remote device configured to produce a telemetric signal that controls the various components of the gastric banding system.

The filling and draining of the band is accomplished by a set of fluidic elements including pumps, valves, and sensors which monitor and/or move fluid between the gastric band and a reservoir. In accordance with various embodiments, different numbers, types, and orientations of the fluidic elements may be utilized to obtain the desired results. Any and/or all of these various components may be configured to be controlled by a remote transmitter.

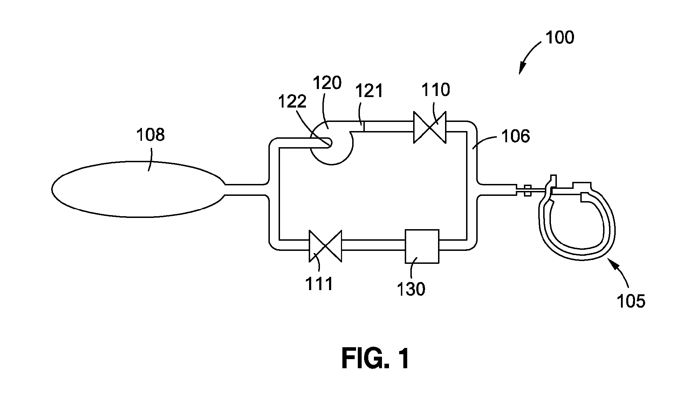

Turning now to FIG. 1, a gastric banding system **100** includes a gastric band **105**, a reservoir **108**, a pump **120**, a sensor module **130** and flow control devices such as valves **110** and **111**. Each of the components of the system **100** is implantable in a patient using conventional surgical techniques. The reservoir **108**, the pump **120**, the valves **110** and **111** and the sensor module **130** may be used to replace or complement a conventional access port for adjusting inflation of the gastric band **105**. In some embodiments, the system **100** includes a conventional access port which can be used, for example, with a hypodermic needle, to fill and drain the gastric band **105**.

The pump **120** and the valves **110** and **111** may move precisely metered volumes of a fluid (e.g., saline, a drug, and/or combinations thereof) from the reservoir **108** through the sensor module **130** into the gastric band **105**. The reservoir **108** may comprise an elastic polymer, a balloon, a rubber container, a silicone container, a collapsible container, a non-elastomeric container, a bellows, and combinations thereof that are configured to contain the fluid.

Moving the fluid into the gastric band **105** causes inflation of at least one bladder, or inflatable member of the gastric band **105** and constricts around the cardia, or upper portion of the stomach, forming a stoma that restricts the passage of food into a lower portion of the stomach. This stoma can provide a patient with a sensation of satiety or fullness that discourages overeating. In contrast, moving the fluid out of at least one inflatable member of the gastric band **105** contracts the pressure around the cardia and allows a stoma to be at least partially released and regains the patient's hunger sensation.

The valves **110** and **111** can be any flow control device to allow precise delivery of fluid and precise flow rates through the valves in response to inductive powering signals received from a remote transmitter. For example, flow control devices as disclosed herein may comprise piezoelectric valves, solenoid valves, membranes, tubes, pressure regulators, ultrasonic flow meters, thermal mechanisms, mass flow sensors, turbines, paddle wheels and combinations thereof.

The pump **120** may comprise any device for moving fluid through a system. For example, the pump **120** may comprise a piezoelectric motor, an electromagnetic motor, an AC motor, a DC motor, a stepper motor and combinations thereof.

Although "transmitter" may be used herein, in should be understood that the remote transmitter may also be a wireless receiver and/or transceiver operable to take readings from the flow sensing module **130** to determine the amount of fluid entering and/or exiting the gastric band **105**, and/or to send or receive other types of information associated with the gastric banding system **100**.

In various embodiments, the remote transmitter provides access to system data and functions and is an external, handheld, reusable battery-powered device. The remote transmitter can be made of any rugged plastic material including, polypropylene, cyclicolephin co-polymer, nylon, and other compatible polymers and the like. Further, the remote transmitter has a user interface including at least one display and at least one user input. The remote transmitter permits a clinician or a patient to navigate through menu driven screens used for data entry, data collection, and control of gastric banding system **100**.

The remote transmitter is capable of communicating with the gastric banding system **100**. "Capable of communicating" as used herein refers to the remote transmitter's ability to establish communications with the gastric banding system **100**, yet still have the ability to break communication and the systems described herein still function. To establish communication, in one example embodiment, once the remote transmitter is initialized, a display shows a searching query for a nearby gastric banding system **100**. As the remote transmitter is brought into range of the gastric banding system **100**, the display shows the strength of the communication link. Once stable communications have been acquired, the display shows the serial number (or other unique patient data) of the system so a clinician can verify they have the appropriate patient records in hand. If the patient requires a tightening of the gastric band **105**, the clinician can enter the amount of the desired volume increase. The remote transmitter can also display the current volume within the gastric band **105** and indicate the new volume as the gastric band **105** fills. The remote transmitter can also indicate desired and actual volumes during the gastric band **105** draining.

In accordance with various embodiments, the gastric banding system **100** allows for a remotely controlled adjustment without needles, non-invasively, by using the remote transmitter. When compared to conventional gastric banding systems having standard access ports which exclusively require syringe access, the presently described systems and apparatus offer several benefits. First, for conventional access ports located under a thick layer of fatty tissue, which is generally the case as the devices are generally used to treat obesity, the access port can be difficult to locate. The present systems reduce or eliminate the need for port location as the use of the remote transmitter removes the necessity of adjustment using a syringe.

Secondly, accessing the access port in conventional systems, when there is ambiguity on its location, can cause damage by accidentally puncturing the tubing which connects the access port to the gastric band. This damage can require another surgery in order to repair the punctured tubing. Further, when a conventional access port cannot be located by palpation, x-ray imaging may be required to guide a needle into the access port. Such imaging practices put a patient at risk for x-ray radiation exposure. The present systems and apparatus remove the need for these unnecessary procedures and save the patient from x-ray radiation exposure. As described herein, the present systems and apparatus are compatible with magnetic resonance imaging (MRI), which is much safer for a patient.

The fluids used within the systems include any fluid that is biocompatible and incompressible. The fluid has no adverse effect on the patient in the unlikely event that a leak emanates from the system. The fluid can simply be water or any biocompatible polymer oil such as caster oil. In an example embodiment, the fluid is saline, a drug, and/or combinations thereof.

The tubing **106** connects the various components of the system **100** and comprises any biocompatible flexible tubing that does not degrade *in vivo.* The tubing **106** is configured to withstand hydraulic forces up to hundreds of psi without leakage. This hydraulic pressure tolerance is true of the entire fluid path of the systems described herein. Although the systems described herein do not generally leak, if they do, fluid is generally lost at a rate less than about 0.2cc/yr, for example, between about 0.01 to 0.07 cc/yr.

According to various embodiments, components of the gastric banding system **100** may be placed in their respective positions within a patient using common surgical techniques. The surgical techniques may be similar to those used in the placement of conventional gastric banding systems. For example, the gastric band **105** may be placed around the stomach using laparoscopic techniques, as known to those of skill in the art. Like a conventional access port, various components of the gastric banding system **100** may be sutured onto the rectus muscle sheath or any other conveniently accessible muscle. The tubing **106** to the gastric band **105** passes through the rectus muscle into the peritoneal cavity in the same manner as the tubing of a conventional access port.

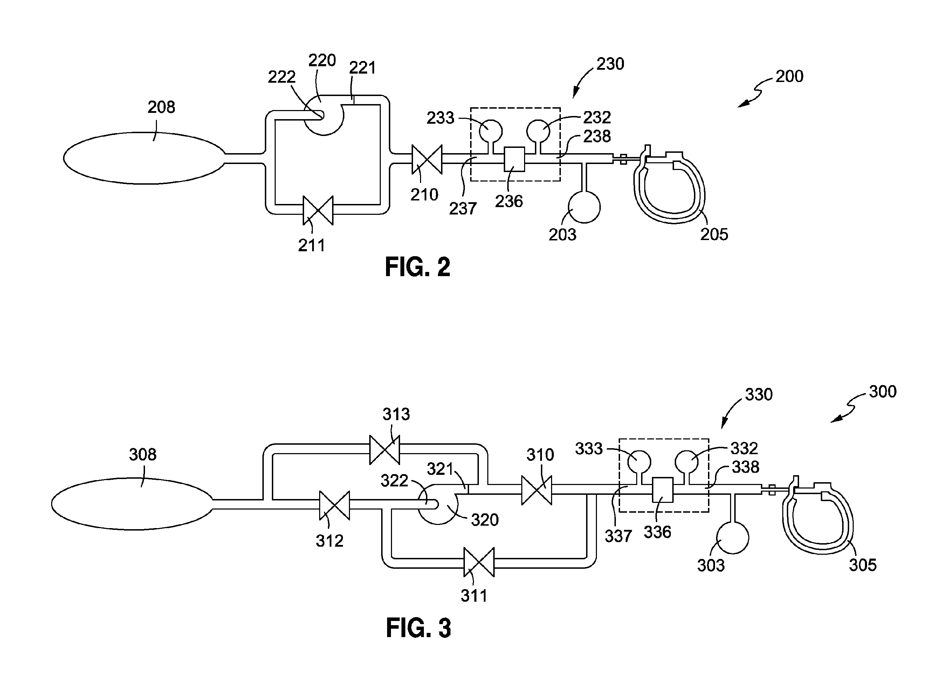

FIG. 2 illustrates an embodiment of the gastric banding system **200** which comprises a valve **211** and a pump **220** connected in parallel to each other. The parallel combination of the valve **211** and the pump **220** is connected in serial with a reservoir **208** on one end and with a valve **210** on the other end. The sensor module **230** is connected in serial with the valve **210** and is disposed between the valve **210** and the gastric band **205**.

The sensor module **230** comprises two pressure sensors, flow meters **232** and **233**, and/or other devices for measuring a desired property or parameter of the fluid in the gastric banding system **200**. For example, the sensor module **230** may be configured to measure flow speed/rate, pressure, fill volume, stress, strain, linear position and combinations thereof.

A calibrated orifice **236** is disposed between the flow meters **232** and **233**. The sensor module **230** comprises a first inlet/outlet **237** and a second inlet/outlet **238**. Because fluid may flow in both directions within the gastric banding system **200**, the inlets/outlets **237** and **238** may alternately function as inlets and outlets. The valve **210** is coupled to the inlet/outlet **237** for controlling the fluid flow into or out of the gastric band **205**.

Although a remote transmitter may be utilized to control various components of the gastric banding system **200**, should the remote transmitter be unavailable, damaged, out of power, or in the event of an emergency, an adjustment of the gastric band **205** may be performed invasively using a needle. For example, an access port **203** (commonly used in other gastric banding systems) may be included in the gastric banding system **205**, in addition to the other components illustrated in FIG. 2. The access port may be located between the valve **210** and the gastric band **205**.

Thus, a clinician may choose to use a standard needle for adjustments, for example, if any of the electronics associated with the gastric banding system **200** become inoperable. Even if the electronics are unavailable, the access port would be available to adjust the gastric band **205**. In the unlikely event that the access port is used, it may be located away from the tubing connection to the gastric band **205** to reduce the potential for tubing needle sticks. Information regarding hydraulically adjustable gastric banding systems including subcutaneous fluid access ports/injection ports may be found in Vincent, U.S. Patent No. 5,601,604; Kusmack, U.S. Patent No. 5,226,429; Birk, U.S. Patent Application Publication No. 2005/0192531.

In embodiments as illustrated in FIGS. 2 and 3, it is possible to measure the fluid flow rate during the process of pumping. The pulsatile flow of pumps may produce a quickly varying pressure wave which can affect the accuracy of pressure and flow measurements. This high pressure gradient can be reduced and the measurement accuracy can be increased by rearranging existing elements and placing additional elements within the flow path such as a valve membrane, soft tubing, or a specially designed fluidic pressure regulator.

Various embodiments of the present invention are configured to speed up the medical procedure of increasing the volume or pressure within a gastric band. The various configurations also facilitate decreasing the time required to perform a partial or complete draining of the gastric band. Thus, embodiments of the present invention provide an implantable device that has an increased efficiency of filling and draining the inflatable portion of the gastric band, with respect to existing implantable pumps.

With continued reference to FIG. 2, in an embodiment, the flow sensing module **230** functions both in pumping and draining the gastric band **205** to facilitate measuring the fluid flow rate in both directions. For example, the flow sensing module **230** may comprise the pressure sensors **232** and **233** positioned on opposite sides of the calibrated orifice **236**. When the calibrated orifice **236** is intended to operate during both the filling and draining process, the calibrated orifice **236** selected may be designed to accurately control bidirectional fluid flow. In various embodiments, the flow sensing module **230** and/or the gastric banding system **200** may include ultrasonic flow meters, thermal mechanisms such as mass flow sensors, or mechanical systems such as turbines and paddle wheels.

An embodiment as illustrated in FIG. 2 allows the pump **220** to "warm up" or stabilize before proceeding with the transfer of fluid between the reservoir **208** and the gastric band **205**. For example, the valve **211** connects a pump outlet **221** to a pump inlet **222**, and the valve **211** may be opened while the valve **210**, to the flow sensing module **230** and the gastric band **205**, remains closed. Opening the valve **211** in this manner allows the pump **220** to stabilize. After stabilization, the valve **210** may be opened. Allowing the pump **220** to stabilize before transferring fluid through the flow sensing module **230** facilitates more accurate measurements of the flow conditions of the fluid. Similarly, in an embodiment as illustrated in FIG. 3, the valves **312** and **313** may be opened before the valves **310** and **311** are opened so that the pump **320** may stabilize before the fluid is directed through the flow sensing module **330** and into the gastric band **305**. The valves **312** and **313** may be configured to withstand approximately 30 psi.

With reference to FIG. 3, an embodiment of the gastric banding system **300** may be configured to modify the drain rate of the gastric band **305** using the passive and active properties of the pump **320**. For example, opening valves **310** and **312** while keeping valves **311** and **313** closed, causes fluid to flow from the gastric band **305** through the deenergized pump **320** to the reservoir **308**. This fluid path through the deenergized pump **320** significantly reduces the drain rate from the gastric band **305**. Additionally, by opening valves **311** and **313** while keeping valves **310** and **312** closed, and directing the drainage through the deenergized pump **320** in a different direction, the drain rate can be slightly reduced since the one-way valves favor this direction of flow.

Further, opening valves **311** and **313** while keeping valves **310** and **312** closed, and supplying voltage to the pump **320**, the drain rate can be slightly increased. As the voltage applied to the pump **320** is increased, the drain flow rate from the gastric band **305** to the reservoir **308** is also increased. This property may be advantageous, for example, when the gastric band **305** contains too little pressure to drain at a desired rate.

In accordance with various embodiments, for example, as illustrated in FIGS. 2 and 3, the gastric banding systems **200** and **300** are configured to obtain an overall lower leak rate because of their arrangement. For designs where the flow sensing module **230** and **330** is determined to contribute to the system leak rate, the arrangement may be adjusted by placing a valve between the flow sensing modules **230** and **330** and the gastric bands **205** and **305**. Such embodiments facilitate reducing the flow rate from the various pumps because the fluid is pumped through the flow resistance caused by the flow sensing modules **230** and **330**.

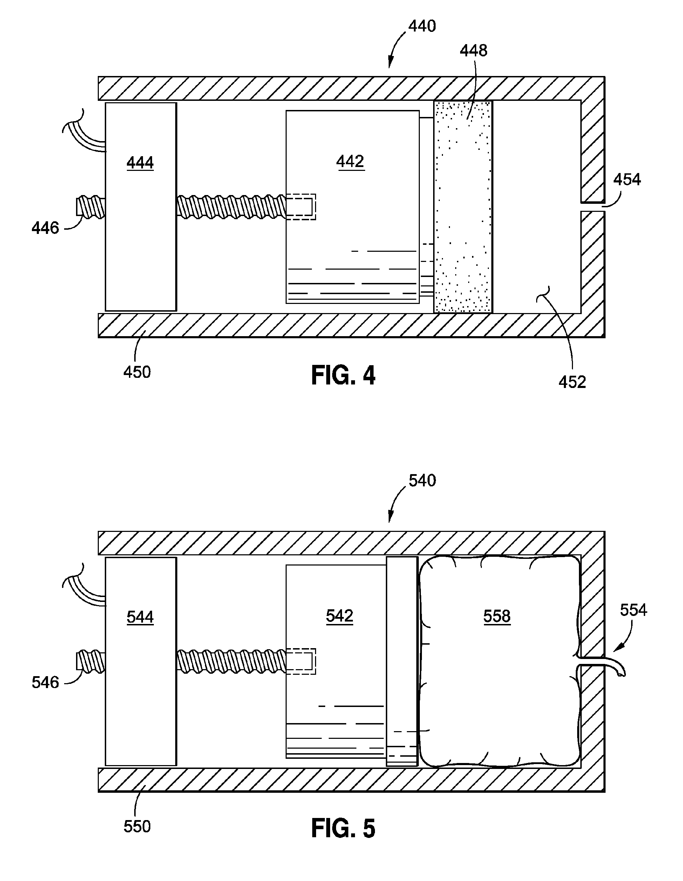

In various embodiments, certain components of the gastric banding system may be combined into a single device. With reference to FIG. 4, one embodiment comprises an implantable pump **440** that includes an integrated chamber or reservoir **452** configured to contain a fluid. The reservoir **452** may comprise an elastic polymer, a balloon, a rubber container, a silicone container, a collapsible container, a non-elastomeric container, a bellows, and combinations thereof that are configured to contain the fluid. The pump **440** further comprises a motor **444** that drives a piston **442** via a leadscrew **446** to cause fluid to exit and/or enter a chamber **452** via a fluid port **454**. Thus, the pump **440** may be referred to herein as an implantable, motorized piston pump. It should be understood that the leadscrew **446** may comprise a screw, a rod, combinations thereof and/or other structure capable of driving the piston **442** using the motor **444**. For example, the leadscrew **446** may comprise a smooth portion and a textured portion to facilitate moving the piston **442** by translational movement.

Although the pump **440** may be utilized in connection with filling and draining a gastric band, it should be understood that the pump **440** has other uses contemplated within the scope of this disclosure. For example, there are many medical situations where the pump **440** may be employed, where it is useful to move the fluid from one place within the body to another place within the body, such as in drug delivery and the modulation of artificial sphincters.

The motor **444** uses electrical energy to turn a gear which moves the leadscrew **446** back and forth to move the piston **442**. The piston **442** and a seal **448** are tightly coupled to and housed within a block **450**. The space bounded by the piston **442** and the interior of the block **450** forms a chamber **452**, the volume of which contracts and expands as the piston **442** moves within the block **450**.

Various types of motors may be used to drive the piston **442**, each having various electrical requirements, driving forces, driving speeds and compatibility with magnetic resonance imaging. The motor **444** may comprises any system or device capable of developing linear and/or rotary motion. Linear motors such as piezoelectric linear motors or electromagnetic linear motors may be used to directly drive the piston. For example, an armature may be used to move the piston **442**, which does not have the helical shape of the leadscrew **446**. In an embodiment, a non-electromagnetic and/or non-magnetic linear drive motor may be used.

Further, in various embodiments, a rotary drive motor may be geared to provide the desired linear motion. Rotary motors such as AC, DC, stepper, or piezoelectric motors can be used to drive a gear system which, in turn, moves the piston **442**. The gear system may be any style which is capable of converting rotational motion into linear motion, and examples include a leadscrew, a ball screw, a jack screw, a rod, and combinations thereof. In various embodiments, the leadscrew **446** may rotate and/or translate with respect to the outer casing of the motor **444** since the gear system may be located between the motor **444** and the leadscrew **446** or between the leadscrew **446** and the piston **442.**

The piston **442** is configured to use the motion of the leadscrew **446** to apply force on the fluid in the reservoir **452**. The piston **442** may be of any cross-sectional shape, including circular, annular, triangular and/or other shapes. The piston **442** may act directly on the fluid by utilizing a seal **448** and/or a wiper.

With reference to FIG. 5, in one embodiment, a piston **542** may act indirectly on the fluid if it pushes on a container **558** such as an elastic balloon, an inelastic bladder, a bellows, and combinations thereof. If the piston acts on the container **558**, a seal may not be required and/or the piston may act on the container to move the fluid without a seal. However, a seal may be utilized in connection with the container **558**.

With reference to FIGS. 4 and 5, the piston **442** (**542**) and/or the seal **448** may have a flat face or a tapered face depending on the desired flow properties and the available space for pump **440** (**540**). Flow properties and/or device size may also determine if the fluid port **454** (**554**) is placed within the block **450** (**550**) in-line with the center of piston **442** (**542**) or off-axis from the center of piston **442** (**542**). Additionally, as will be discussed further below, fluid port **454** (**554**) may be placed within piston **442** (**542**) to obtain a different set of operating parameters and/or properties.

It should be understood that although the various figures may illustrate a gap and/or space between the piston and the block and/or between the seal and the block, the various components of the pumps disclosed herein are configured to pump a fluid into a gastric band. Thus, the pistons and/or seals are configured to move within the pump in a manner that causes the fluid to be expelled from the reservoir. Tolerances may be tighter or looser than those illustrated in the figures without departing from the scope of the present invention. Further, other tolerances, materials, seals, lubricants, components and/or combinations thereof may be utilized to obtain the desired pumping characteristics. Additionally, in some embodiments, the pump may not include a seal between the piston and the filling reservoir.

With reference again to FIG. 4, in an embodiment, the orientation or configuration of the motor **444** and the piston **442** may affect function and/or size of the pump **440**. For example, when the motor **444** is placed inline with the piston **442**, the load of the piston **442**, which is inline with the motor **444**, produces a symmetrical distribution of forces. An alternate embodiment includes a configuration where the motor **444** is placed off axis to the piston **442**. This configuration results in an asymmetric loading of the piston **442**.

The motor **444**, the gearing, and the leadscrew **446** can be connected to the piston **442** in a way to match certain characteristics to the mechanical system by pulling and/or pushing the piston **442** or to obtain substantially equal pushing and pulling forces. In some motor/piston combinations, a return spring may be utilized to assist the movement of the piston **442** into or out of the block **450**.

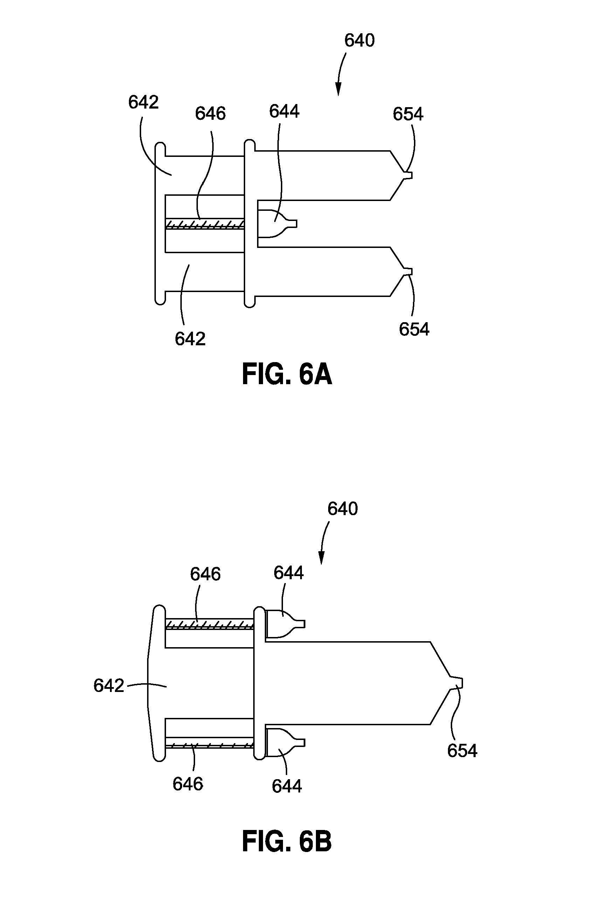

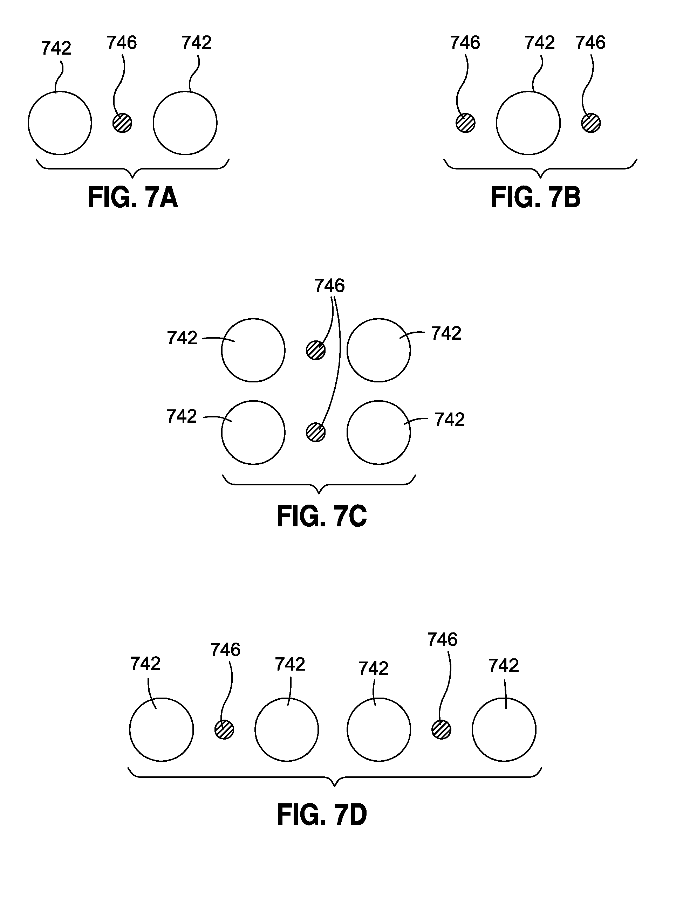

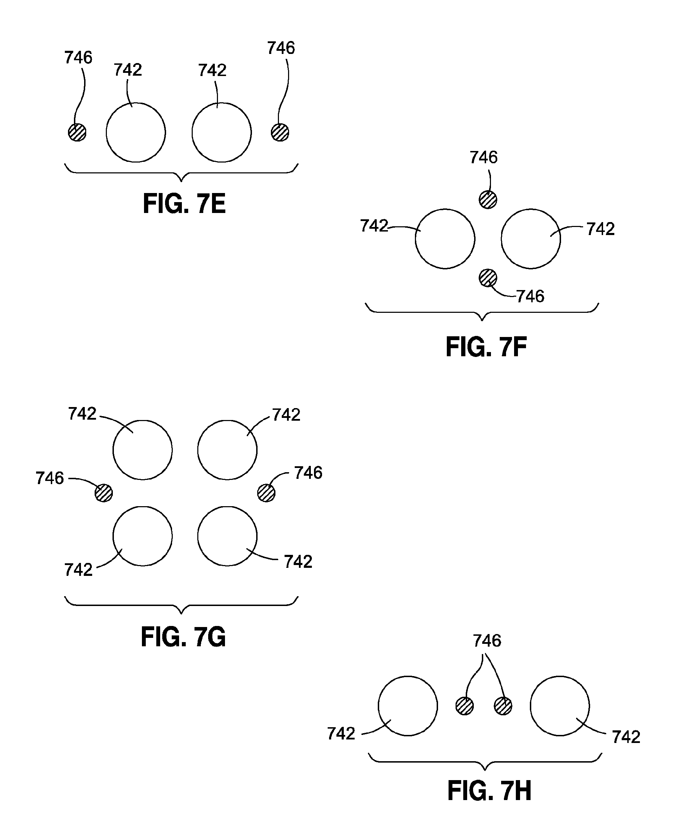

Although FIG. 4 illustrates the pump **440** having one motor and one piston, it should be understood that any number and/or combination of motors and pistons may be utilized in accordance with the present invention. For example, FIG. 6A illustrates an embodiment with two pistons **642** and one motor **644** and one leadscrew **646**. FIG. 6B illustrates an embodiment with one piston **642** and two motors **644** and two leadscrews **646**. FIGS. 7A-7H illustrate various other combinations of the pistons **742** and the leadscrews **746** according to embodiments of the present invention.

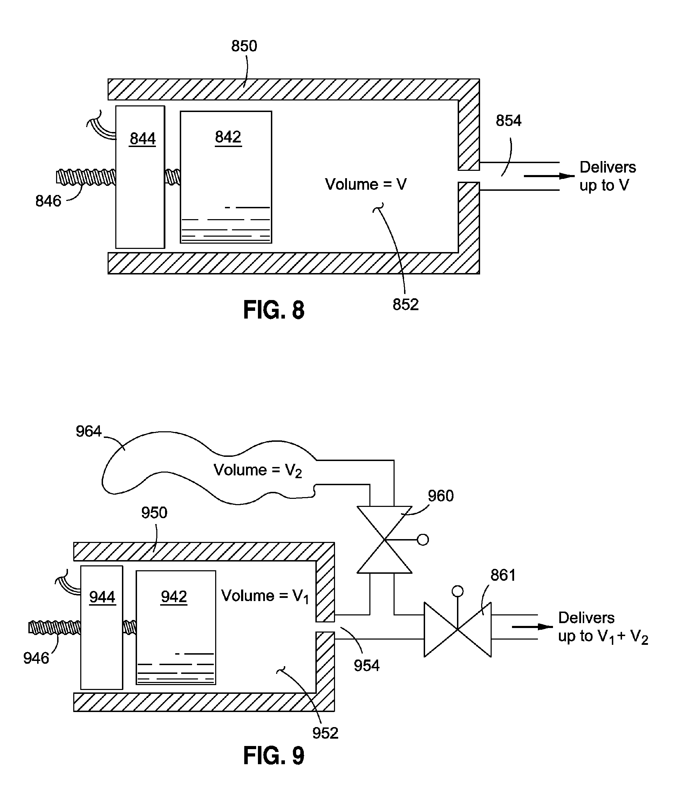

With reference to FIG. 8, in one embodiment, the total volume (V) which is moved by the piston **842** may reside within the reservoir **852**. In this case, the volume pumped by the system may be any part of the total volume V, but not more than V.

However, with reference to FIG. 9, one embodiment facilitates delivering more than the volume (V₁) of fluid contained in the reservoir **952**. For example, flow control devices such as valves **960** and **961** and the external reservoir **964** having a volume (V₂) may be added to the system, and the total amount which can be delivered (e.g., V₁ + V₂) can be greater than the volume of the reservoir **952**. The reservoir **952** and/or the external reservoir **964** may comprise an elastic polymer, a balloon, a rubber container, a silicone container, a collapsible container, a non-elastomeric container, a bellows and combinations thereof that are configured to contain the fluid. In one embodiment, the reservoir **952** has a volume of between approximately 0.1-5 cc (preferably approximately 1 cc), and the external reservoir **964** has a volume of between approximately 1-10 cc (preferably approximately 5 cc).

With the valve **960** open and the valve **961** closed, the piston **942** can reciprocate within the block **950** in order to drain or fill the external reservoir **964**. With the valve **960** closed and the valve **961** open, the piston **942** can reciprocate within the block **950** in order to drain or fill the gastric band. By placing the valves **960** and **961** proximate to the inlet/outlet port **954** and reducing the amount of fluid between the valves and the fully compressed piston **942**, the dead space of the system can be reduced and the efficiency of the system can be improved. By a similar method, or by increasing the stroke volume, the compression ratio (volume pumped in one stroke divided by the dead space volume) can be increased.

In accordance with various embodiments, the motorized piston pump system is reversible. For example, with reference to FIG. 8, when the total volume is contained within the reservoir **852**, fluid may be driven out of the reservoir **852** by driving the piston **842** through the block **850** toward the inlet/outlet **854**, and the fluid can be returned into the reservoir **852** by pulling the piston **842** out of the block **850** away from the inlet/outlet **854**.

With reference to FIG. 9, reversing the flow of fluid may also be accomplished. The valves **960** and **961** and the piston **942** may be sequenced, as discussed above, so that the fluid flows into or out of the reservoir **952**.

Although various embodiments disclosed herein comprise a motor that is located outside of the fluid path which it is used to pump (see, e.g., FIG. 4 where piston **442** pumps the fluid in the reservoir **452**). In an embodiment, the motor may be configured to function within a fluid environment, and may be positioned in contact with or surrounded by the fluid.

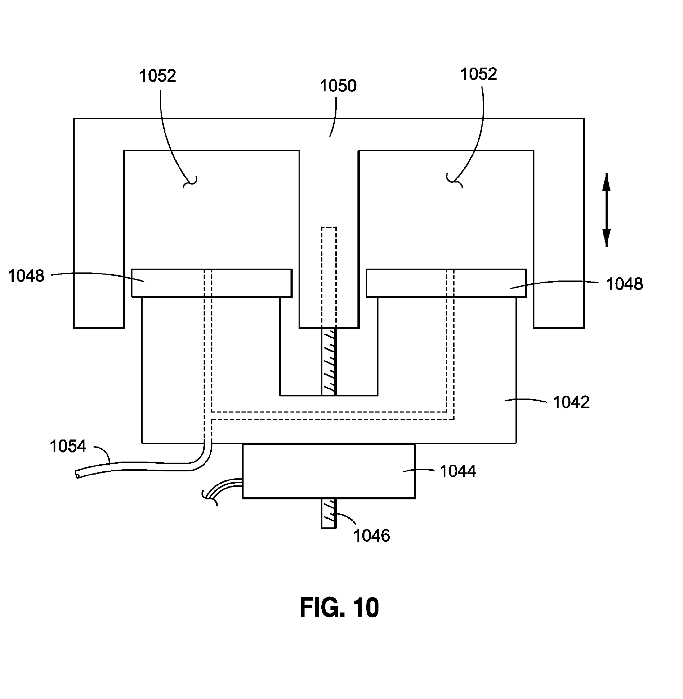

With reference to FIG. 10, in one embodiment, the inlet/outlet port **1054** may be placed within the piston **1042**, as compared to within the block **950** as illustrated in FIG. 9. The location of the port within the piston or the block may be determined by certain performance requirements. For example, if the fluid connection(s) is/are routed out of the same assembly that holds the motor, both the fluid connection(s) and the electrical connection(s) to the motor can remain stationary and be less susceptible to bending fatigue, as illustrated in FIG. 10.

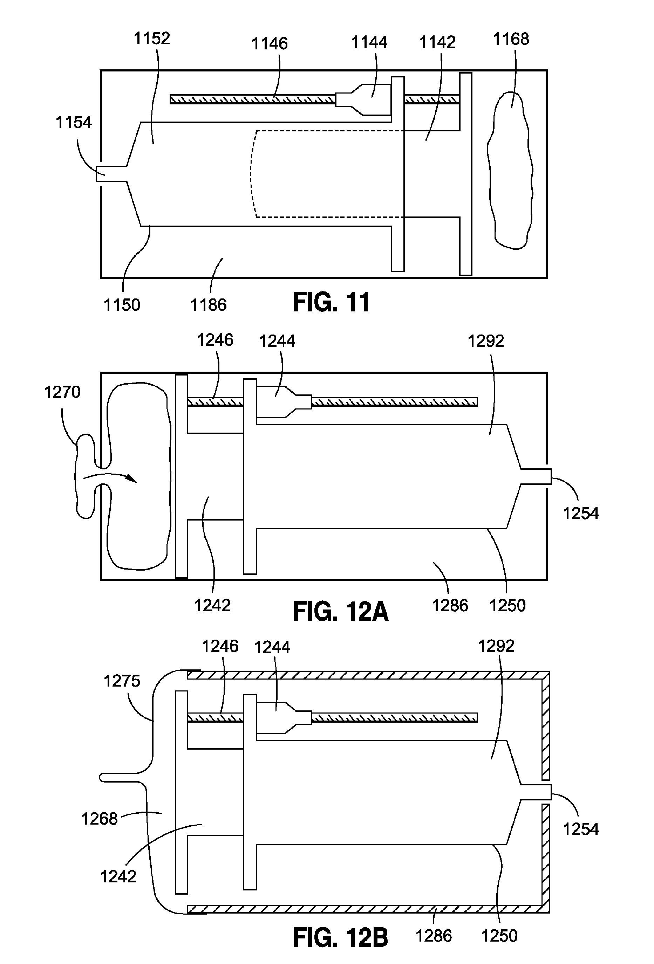

Turning now to FIG. 11, when an internal element (such as a piston **1142**) moves within a casing **1186**, the friction or resistive forces may be reduced by interposing low friction or lubricious materials between the moving surfaces. Additionally, the friction may be reduced by including fluid- or air-filled spaces between the moving surfaces of the pump and casing components.

As the piston **1142** moves within the enclosed, sealed casing **1186** and the block **1150** and ejects fluid from the reservoir **1152**, the overall volume of the system decreases. However, because the casing **1186** is a rigid housing with a fixed volume, the discrepancy results in a mismatched vacuum in vacuum area **1168** of the casing **1186**. In order to compensate for this volume/vacuum mismatch condition, various embodiments comprise one or more of a flexible housing, an internal equalizer, or a reduced volume change in the piston block **1150** and/or in casing **1186**. For example, a rigid housing may be converted to a flexible housing and then any changes in the internal volume will cause corresponding volume changes within the housing.

FIGS. 12A-12G illustrate various embodiments for adapting an external housing **1250** to include flexible structures. The flexible structures can be constructed either from metal or polymeric material. For example, FIG. 12A illustrates a volume transfer bladder **1270** that is partially located within and without the casing **1286**. As the piston **1242** moves in the block **1250**, the volume transfer bladder **1270** enters or exits the casing **1286** to compensate for the change in volume. Similarly, as illustrated in FIG. 12B, part of the casing **1286** may comprise a flexible portion **1275** that expands and contracts as the piston **1242** moves within the block **1250**.

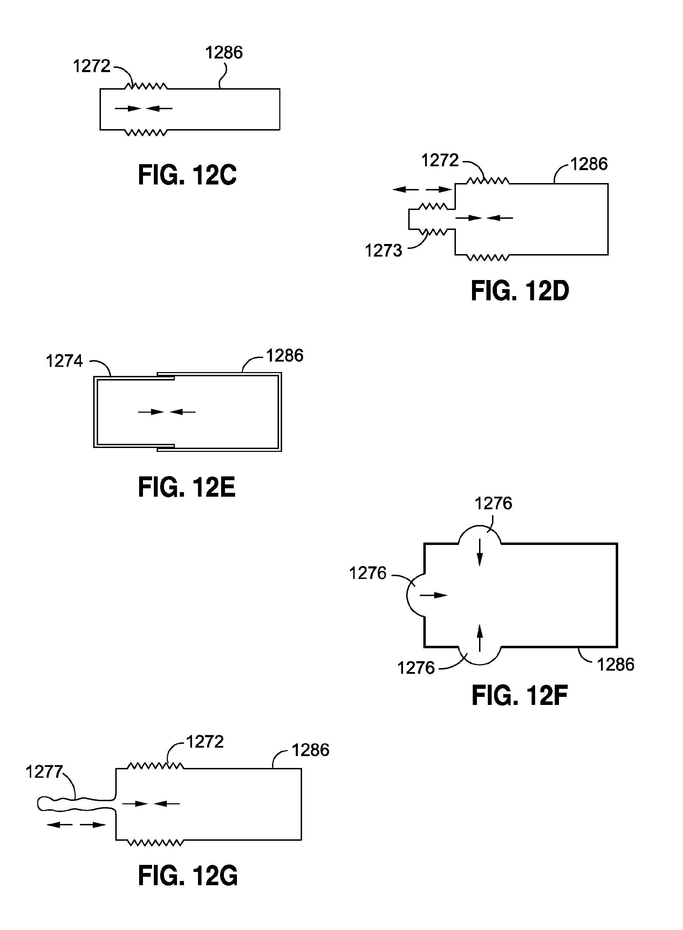

With reference to FIG. 12C, in one embodiment, the overall length of the casing **1286** may be configured to change to compensate for the change in volume. For example, a first set of bellows **1272** may be utilized to facilitate the change in length of the casing **1286**. With reference to FIG. 12D, the first set of bellows **1272** may be used in conjunction with a second set of bellows **1273** that moves in opposition to the first set of bellows **1272**. Such a configuration allows for compensation of a volume change in the casing **1286** while maintaining a fixed length for the casing **1286**. Furthermore, with reference to FIG. 12G, the first set of bellows **1272** may be utilized in connection with an elongating balloon **1277** that extends from or retracts into the casing **1286** depending on the motion of the piston.

With reference to FIG. 12E, one embodiment includes a sliding segment **1274** that slides with respect to the casing **1286** in response to motion of the piston. Such motion allows the volume of the casing **1286** to change as fluid exits or enters the reservoir of the pump. Utilizing sliding components may improve the system's resistance to fatigue and failure. However, in some embodiments, for example, with reference to FIG. 12F, the casing **1286** may comprise at least one flexible segment **1276** that expands or contracts in response to motion of the piston.

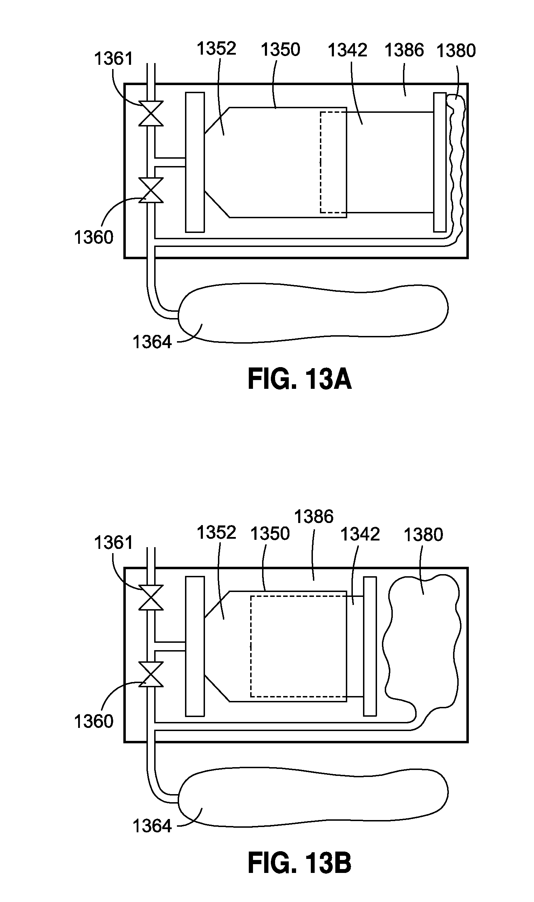

In accordance with another embodiment, and with reference to FIGS. 13A-13B, an internal equalizer mechanism may be used to compensate for a change in volume. For example, the casing **1386** may comprise a bladder **1380** with a volume that increases or decreases as the piston **1342** moves within the block **1350**. The bladder **1380** may be utilized with or without an external reservoir **1364**. The valves **1360** and **1361** may be alternately opened or closed to control the volume of fluid within the reservoir **1352** and/or the external reservoir **1364**, as discussed above with respect to FIG. 9, to compensate for volume changes due to movement of the piston **1342**. When the valve **1361** is opened, fluid may be expelled from the reservoir **1352**, and the volume change caused by movement of the piston **1342** causes fluid from the external reservoir **1364** to be drawn into the bladder **1380** (see FIG. 13B). Then, if more fluid is desired within the reservoir **1352**, the valve **1361** may be closed and the valve **1360** may be opened to allow fluid to be drawn from the bladder **1380** and/or the external reservoir **1364**.

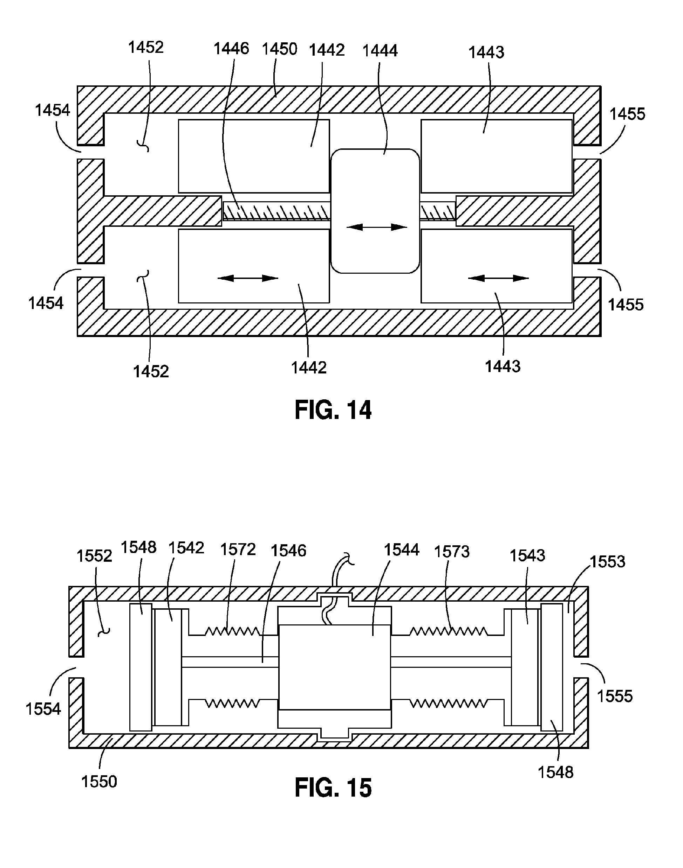

Another embodiment configured to compensate for the vacuum/pressure mismatch is illustrated in FIG. 14, for example, to minimize the parasitic effects of a vacuum. Such an embodiment comprises multiple, opposing pistons **1442** and **1443** that are configured to reduce the volume/pressure mismatch by minimizing and/or eliminating the amount of volume change in the motor piston pump. For example, as the piston **1442** moves toward the inlet/outlet **1454**, the piston **1443** moves away from the inlet/outlet **1455** to compensates for the volume change induced by the movement of the piston **1442**. By arranging two or more pistons **1442** and **1443** to act oppositely for a given motor **144** stroke (e.g., one pumping and one filling), a nearly continuous pumping action may be achieved. The inlet/outlet **1455** may be connected to an external reservoir as illustrated in FIGS. 13A-13B, and may operate in conjunction with various valves (also as illustrated in FIGS. 13A-13B) to facilitate movement of the fluid within the system. The opposing pistons **1442** and **1443** facilitate reducing the overall size of the implantable device.

With reference to FIG. 15, one embodiment comprises sealed bellows **1572** and **1573** that expand and contract in response to movement of the pistons **1542** and **1543**. The bellows **1572** and **1573** may be constructed of metal or any other material that facilitates operation of the pump as disclosed herein. By enclosing the motor **1544** and the leadscrew **1546** within the bellows **1572** and **1573**, the rotating parts and electronics may be hermetically sealed. When two opposing bellows **1572** and **1573** are moved opposite of one another (i.e., one compresses as the other expands), the pressure of the gas or fluid within the sealed system can be kept substantially constant. The seals **1548** (and/or bladders as discussed with respect to FIG. 5-flexible bladder **558**) may be used to further seal the motor **1544** and/or other electronics or components of the piston pump.

## Claims

1. An implantable device (100, 200) configured for filling and draining an inflatable portion of a gastric band (105, 205), the implantable device comprising:
a reservoir (108, 208) for holding a fluid;
a sensor (130, 230) positioned between the gastric band and the reservoir, the sensor having a first inlet (237) and a second inlet (238), the fluid flowing from the first inlet to the second inlet when filling the inflatable portion of the gastric band, the fluid flowing from the second inlet to the first inlet when draining the inflatable portion of the gastric band, the sensor monitoring a parameter of the fluid when filling and draining the inflatable portion of the gastric band;
a first flow control device (110, 210), coupled to the first inlet of the sensor, for controlling a flow of the fluid when filling and draining the inflatable portion of the gastric band; and
a pumping device (120, 220), coupled between the reservoir and the first flow control device, for moving the fluid into and out of the inflatable portion of the gastric band, the pumping device capable of being activated and deactivated using a telemetric signal received from a remote device,
**characterized in that** the sensor includes two pressure sensors (232, 233) and a calibrated orifice (236) positioned fluidly between the two pressure sensors.

2. The implantable device of Claim 1 wherein the first flow control device (110, 210) is selected from a group consisting of a valve, a membrane, a tube, a pressure regulator, an ultrasonic flow meter, a thermal mechanism, a mass flow sensor, a turbine, a paddle wheel, and combinations thereof.

3. The implantable device of any one of the preceding Claims further comprising a second flow control device coupled to the second inlet of the sensor for controlling movement of the fluid when draining the gastric band.

4. The implantable device of Claim 3 wherein the second flow control device is selected from a group consisting of a valve, a membrane, a tube, a pressure regulator, an ultrasonic flow meter, a thermal mechanism, a mass flow sensor, a turbine, a paddle wheel, and combinations thereof.

5. The implantable device of any one of the preceding Claims wherein the pumping device (120, 220) is selected from a group consisting of a pump, a piezoelectric motor, an electromagnetic motor, an AC motor, a DC motor, a stepper motor, and combinations thereof.

6. The implantable device of any one of the preceding Claims wherein the parameter is selected from a group consisting of a flow speed, a pressure, a fill volume, a stress, a strain, a linear measurement, and combinations thereof.

7. The implantable device of any one of the preceding Claims wherein the reservoir (108, 208) is selected from a group consisting of an elastic polymer, a balloon, a rubber container, a silicone container, and combinations thereof.

8. The implantable device of Claim 3 further comprising an access port (203) fluidly coupled between the second flow control device and the inflatable portion of the gastric band.

9. The implantable device of any one of the preceding Claims wherein the fluid is selected from a group consisting of a drug, a saline solution, and combinations thereof.

10. The implantable device of any one of the preceding Claims wherein the pumping device (120, 220) comprises a non-magnetic electrical drive.

## Patentansprüche

1. Implantierbare Vorrichtung (100, 200), die zum Füllen und Leeren eines aufblasbaren Abschnitts von einem Magenband (105, 205) eingerichtet ist, wobei die implantierbare Vorrichtung aufweist:
ein Reservoir (108, 208) zum Halten eines Fluids;
einen Sensor (130, 230), der zwischen dem Magenband und dem Reservoir positioniert ist, wobei der Sensor einen ersten Einlass (237) und einen zweiten Einlass (238) aufweist, das Fluid von dem ersten Einlass zu dem zweiten Einlass fließt, wenn der aufblasbare Abschnitts des Magenbands gefüllt wird, das Fluid von dem zweiten Einlass zu dem ersten Einlass fließt, wenn der aufblasbare Abschnitt des Magenbands geleert wird, und der Sensor einen Parameter des Fluids überwacht, wenn der aufblasbare Abschnitt des Magenbands gefüllt und geleert wird;
eine erste Strömungssteuereinrichtung (110, 210), die mit dem ersten Einlass des Sensors zum Steuern eines Fluidstroms gekoppelt ist, wenn der aufblasbare Abschnitt des Magenbands gefüllt und geleert wird; und
eine Pumpeinrichtung (120, 220), die zwischen dem Reservoir und der ersten Strömungssteuereinrichtung zum Bewegen des Fluids in den aufblasbaren Abschnitt des Magenbands und aus ihm heraus verbunden ist, wobei die Einrichtung imstande ist, unter Verwendung eines telemetrischen Signals, das von einer Ferneinrichtung empfangen wird, aktiviert und deaktiviert zu werden,
**dadurch gekennzeichnet, dass** der Sensor zwei Drucksensoren (232, 233) und eine kalibrierte Öffnung (236) aufweist, die zwischen den zwei Drucksensoren in Fluidverbindung positioniert ist.

2. Implantierbare Vorrichtung nach Anspruch 1, bei der die erste Strömungssteuereinrichtung (110, 210) aus einer Gruppe ausgewählt ist, die aus einem Ventil, einer Membran, einem Rohr, einem Druckregler, einem Ultraschallströmungsmessgerät, einem Wärmemechanismus, einem Massenstromsensor, einer Turbine, einem Schaufelrad und Kombinationen daraus besteht.

3. Implantierbare Vorrichtung nach einem der vorstehenden Ansprüche, ferner mit einer zweiten Strömungssteuereinrichtung, die mit dem zweiten Einlass des Sensors zum Steuern einer Bewegung des Fluids beim Entleeren des Magenbands gekoppelt ist.

4. Implantierbare Vorrichtung nach Anspruch 3, bei der die zweite Strömungssteuereinrichtung aus einer Gruppe ausgewählt ist, die aus einem Ventil, einer Membran, einem Rohr, einem Druckregler, einer Ultraschallströmungsmesseinrichtung, einem Wärmemechanismus, einem Massenstromsensor, einer Turbine, einem Schaufelrad und Kombinationen daraus besteht.

5. Implantierbare Vorrichtung nach einem der vorstehenden Ansprüche, bei der die Pumpeinrichtung (120, 220) aus einer Gruppe ausgewählt ist, die aus einer Pumpe, einem piezoelektrischen Motor, einem elektromagnetischen Motor, einem Wechselstrommotor, einem Gleichstrommotor, einem Schrittmotor und Kombinationen daraus besteht.

6. Implantierbare Vorrichtung nach einem der vorstehenden Ansprüche, bei welcher der Parameter aus einer Gruppe ausgewählt ist, die aus einer Strömungsgeschwindigkeit, einem Druck, einem Füllvolumen, einer Spannung, einer Dehnung, einer linearen Messung und Kombinationen daraus besteht.

7. Implantierbare Vorrichtung nach einem der vorstehenden Ansprüche, bei der das Reservoir (108, 208) aus einer Gruppe ausgewählt ist, die aus einem elastischen Polymer, einem Ballon, einem Kautschukbehälter, einem Silikonbehälter und Kombinationen daraus besteht.

8. Implantierbare Vorrichtung nach Anspruch 3, ferner mit einer Zugangsöffnung (203), die zwischen der zweiten Strömungssteuereinrichtung und dem aufblasbaren Abschnitt des Magenbands in Fluidverbindung ist.

9. Implantierbare Vorrichtung nach einem der vorstehenden Ansprüche, bei der das Fluid aus einer Gruppe ausgewählt ist, die aus einem Wirkstoff, einer Salzlösung und Kombinationen daraus besteht.

10. Implantierbare Vorrichtung nach einem der vorstehenden Ansprüche, bei der die Pumpeinrichtung (120, 220) einen nichtmagnetischen elektrischen Antrieb aufweist.

## Revendications

1. Dispositif implantable (100, 200) configuré pour remplir et drainer une partie gonflable d'un anneau gastrique (105, 205), le dispositif implantable comprenant :
un réservoir (108, 208) pour contenir un fluide ;
un capteur (130, 230) positionné entre l'anneau gastrique et le réservoir, le capteur ayant une première entrée (237) et une deuxième entrée (238), le fluide circulant depuis la première entrée vers la deuxième entrée lors du remplissage de la partie gonflable de l'anneau gastrique, le fluide circulant depuis la deuxième entrée vers la première entrée lors du drainage de la partie gonflable de l'anneau gastrique, le capteur surveillant un paramètre du fluide lors du remplissage et du drainage de la partie gonflable de l'anneau gastrique ;
un premier dispositif de réglage de débit (110, 210), couplé à la première entrée du capteur, pour régler un débit du fluide lors du remplissage et du drainage de la partie gonflable de l'anneau gastrique ; et
un dispositif de pompage (120, 220), couplé entre le réservoir et le premier dispositif de réglage de débit, pour déplacer le fluide dans et hors de la partie gonflable de l'anneau gastrique, le dispositif de pompage étant capable d'être activé et désactivé en utilisant un signal télémétrique reçu à partir d'un dispositif à distance,
**caractérisé en ce que** le capteur comporte deux capteurs de pression (232, 233) et un orifice calibré (236) positionné de manière fluide entre les deux capteurs de pression.

2. Dispositif implantable de la revendication 1, dans lequel le premier dispositif de réglage de débit (110, 210) est choisi dans un groupe constitué d'une soupape, d'une membrane, d'un tube, d'un régulateur de pression, d'un débitmètre à ultrasons, d'un mécanisme thermique, d'un capteur de débit massique, d'une turbine, d'une roue à aubes, et de combinaisons de ceux-ci.

3. Dispositif implantable de l'une quelconque des revendications précédentes, comprenant en outre un deuxième dispositif de réglage de débit couplé à la deuxième entrée du capteur pour commander le déplacement du fluide lors du drainage de l'anneau gastrique.

4. Dispositif implantable de la revendication 3, dans lequel le deuxième dispositif de réglage de débit est choisi dans un groupe constitué d'une soupape, d'une membrane, d'un tube, d'un régulateur de pression, d'un débitmètre à ultrasons, d'un mécanisme thermique, d'un capteur de débit massique, d'une turbine, d'une roue à aubes, et de combinaisons de ceux-ci.

5. Dispositif implantable de l'une quelconque des revendications précédentes, dans lequel le dispositif de pompage (120, 220) est choisi dans un groupe constitué d'une pompe, d'un moteur piézoélectrique, d'un moteur électromagnétique, d'un moteur à courant alternatif, d'un moteur à courant continu, d'un moteur pas à pas et de combinaisons de ceux-ci.

6. Dispositif implantable de l'une quelconque des revendications précédentes, dans lequel le paramètre est choisi dans un groupe constitué d'une vitesse d'écoulement, d'une pression, d'un volume de remplissage, d'une contrainte, d'une déformation, d'une mesure linéaire, et de combinaisons de ceux-ci.

7. Dispositif implantable de l'une quelconque des revendications précédentes, dans lequel le réservoir (108, 208) est choisi dans un groupe constitué d'un polymère élastique, d'un ballonnet, d'un récipient en caoutchouc, d'un récipient en silicone, et de combinaisons de ceux-ci.

8. Dispositif implantable de la revendication 3, comprenant en outre un orifice d'accès (203) couplé de manière fluide entre le deuxième dispositif de réglage de débit et la partie gonflable de l'anneau gastrique.

9. Dispositif implantable de l'une quelconque des revendications précédentes, dans lequel le fluide est choisi dans un groupe constitué d'un médicament, d'une solution saline, et de combinaisons de ceux-ci.

10. Dispositif implantable de l'une quelconque des revendications précédentes dans lequel le dispositif de pompage (120, 220) comprend une commande électrique non magnétique.
